(19)

Europäisches
Patentamt

European
Patent Office

Office européen
des brevets

(11) **EP 3 012 243 A1**

(12) **EUROPEAN PATENT APPLICATION**
published in accordance with Art. 153(4) EPC

(43) Date of publication:
**27.04.2016 Bulletin 2016/17**

(21) Application number: **14813695.5**

(22) Date of filing: **08.07.2014**

(51) Int Cl.:
**C07C 51/275** (2006.01)   **C07C 201/08** (2006.01)
**C07C 55/14** (2006.01)   **C07C 205/05** (2006.01)

(86) International application number:
**PCT/CN2014/081818**

(87) International publication number:
**WO 2014/202031 (24.12.2014 Gazette 2014/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **21.06.2013 CN 201310250015**

(71) Applicant: **Xiangtan University
Xiangtan City, Hunan Province
411105 (CN)**

(72) Inventors:
• **LUO, Hean
Xiangtan City,
Hunan Province 411105 (CN)**

• **YOU, Kuiyi
Xiangtan City
Hunan Province 411105 (CN)**
• **JIAN, Jian
Xiangtan City
Hunan Province 411105 (CN)**
• **ZHOU, Zhongcang
Xiangtan City
Hunan Province 411105 (CN)**
• **LIU, Pingle
Xiangtan City
Hunan Province 411105 (CN)**

(74) Representative: **Hubert, Philippe et al
Cabinet Beau de Loménie
158, rue de l'Université
75340 Paris Cedex 07 (FR)**

(54) **METHOD FOR CO-PRODUCTION OF ADIPIC ACID AND NITROCYCLOHEXANE**

(57)   The present invention relates to a method for coproducing of adipic acid and nitrocyclohexane in high-selectivity. The method of the present invention is to coproduce adipic acid and nitrocyclohexane in one step from cyclohexane by using nitrogen oxides NOx as the oxidant and nitrating agent, with high selectivity and adjustable ratio of adipic acid and nitrocyclohexane, by adjusting the reaction conditions such as ratio of reactants, reaction temperature and reaction pressure, the types and amount of catalysts or inducing agents. The NO produced by the reaction can be recovered and again reacted with oxygen to produce $NO_x$ for recycling.

Figure 1

**Description**

**Field of the Invention**

The present invention relates to a method for coproducing adipic acid and nitrocyclohexane from cyclohexane with high selectivity in one step.

**Background of the Invention**

Adipic acid (ADA), commonly known as hexanedioic acid, is a white solid at room temperature with the formula $(CH_2)_4 (COOH)_2$, and has the molecular weight of 146.1430 and the normal melting point of 152 °C. Adipic acid is a very important industrial material and intermediate, mainly used in the manufacture of nylon 66, plasticizers, lubricating greases, pesticides and adhesives. The method of synthesizing adipic acid mainly comprises two-step oxidation of cyclohexane, two-step method of cyclohexene hydration-oxidation, one-step (molecular oxygen) oxidation of cyclohexane, carbonylation of butadiene, one-step hydrogen peroxide oxidation of cyclohexene, biological oxidation and the like.

Nitrocyclohexane is a colorless oily liquid at normal temperature with the molecular formula $C_6H_{11}NO_2$ and the molecular weight 129.1571. It has a boiling point of 205.5 °C and is insoluble in water and soluble in organic solvents such as ethanol, ethyl ether, chloroform, benzene, etc., and is an important organic solvent and intermediate (for example through hydrogenation to produce cyclohexylamine), but its use is much less extensive than adipic acid at present. If the reduction of nitrocylohexane to cyclohexanone oxime by the partial hydrogenation may be applied in the industry, the market demand for it can be greatly increased.

Adipic acid is generally produced by a two-step oxidation process using cyclohexane as the raw material. Firstly, KA oil (mainly containing a mixture of cyclohexanone and cyclohexanol) is prepared by air oxidation of cyclohexane, and then adipic acid is prepared by nitric acid oxidation using KA oil as the raw material. The single pass conversion and the KA oil yield in the cyclohexane oxidation process are very low, generally 3%-5% and 82%-83%, respectively. The single pass conversion and adipic acid yield in the nitric acid oxidation of KA oil are not high, generally 5% - 12% and 90%-94%, respectively. The consumption of nitric acid is large, 1.3 tons of 68% nitric acid is consumed per a ton of product, and a large amount of waste acid, wastewater, and toxic gases such as CO, NOx and $N_2O$ are produced. It can be seen that the two-step oxidation of cyclohexane has the disadvantages of high energy consumption, big impact on environment, low resource utilization, high production cost, etc., even though the process is well established and widely used in industrial production. Two-step cyclohexene hydration-oxidation is a new industrialized method in recent years, wherein cyclohexene is firstly hydrated to cyclohexanol, and the latter is formed to adipic acid by nitric acid oxidation. The advantages of this method is the high selectivity of the reaction in the first step, but the second step has high energy consumption, large environmental impact, etc., and further cyclohexene is obtained from selective partial hydrogenation of benzene, which involves relatively harsh reaction and separation conditions and high energy consumption.

One-step synthesis of adipic acid by hydrogen peroxide oxidation of cyclohexene is an environmentally friendly process, wherein adipic acid is prepared in one step by catalytically oxidizing cyclohexene using hydrogen peroxide as the oxidant at catalytic conditions. In view of the requirement to use a relatively expensive catalyst such as methyl trioctyl ammonium chloride, sodium tungstate-oxalic acid system or sodium tungstate-sulfuric acid system, the harsh reaction conditions of preparing cyclohexene and separation conditions, the high production costs, high consumption of hydrogen peroxide and other reasons, the industrialization of the one-step process of synthesizing adipic acid by cyclohexene oxidation is hampered. Biological oxidation is to use the enzyme encoded by a gene cluster isolated from an aerobic denitrification bacteria strain, which may convert cyclohexanol into adipic acid at a suitable growth conditions. However, the process is expensive, and is not suitable for large-scale industrial production at present.

The direct oxidation of cyclohexane to adipic acid using molecular oxygen is a long-anticipated process, and early in the 1940s, it was proposed in US 2223493 (1940) that adipic acid is directly synthesized by air oxidation of cyclohexane using acetic acid as the solvent and soluble transition metal (cobalt, copper, manganese, etc.) salt as the catalyst. Based on this method, US 4263453 (1981) disclosed that the conversion rate of cyclohexane and the selectivity of adipic acid may be improved by increasing the amount of acetic acid solvent (the molar ratio of cyclohexane and acetic acid is 1: 6) and introducing a small amount of butanone and water; US 5321157 (1994) disclosed that the amount of acetic acid solvent may be reduced (the molar ratio of cyclohexane and acetic acid is close to 1: 1) and higher adipic acid selectivity may be obtained, by using oxygen-enriched air. Although these methods can obtain a higher conversion rate of cyclohexane and a higher selectivity of adipic acid, there are many problems: firstly, acetic acid has a severe corrosion on the equipments at the reaction temperature and pressure conditions; secondly, the desired product adipic acid and other byproducts are difficult to be separated and purified from acetic acid solvent; and thirdly, soluble catalyst is difficult to be separated from acetic acid solvent. Although FRA2722783 (1996) and FRA2746671 (1997) proposed a method to solve the catalyst recycling, the process is complicated and expensive. Therefore, the process for preparing adipic acid by direct oxidation of cyclohexane with molecular oxygen using acetic acid as solvent is difficult to industrialization.

US0147777A1 (2004) disclosed a method of synthesizing cyclohexanol /cyclohexanone and adipic acid by air oxidation of cyclohexane using caprylic acid as solvent, cobalt acetylacetonate as catalyst, a compound containing imide groups as cocatalyst. US7507856B2 (2009) disclosed a method of synthesizing adipic acid by air oxidation of cyclohexane using 4-tert-butyl-benzoic acid as a solvent, manganese acetylacetonate as a catalyst, cyclohexanone as an inducing agent, wherein the conversion of cyclohexane and adipic acid selectivity were up to 7.17% and 53.6%, respectively. US 0095258 A1 (2012) disclosed a method of synthesizing adipic acid by molecular oxygen oxidation of cyclohexane using acetonitrile as a solvent, a noble metal (Au, Pd, Pt, etc.) supported on metal oxide ($TiO_2$, $ZrO_2$, MgO, etc.) as a catalyst, wherein cyclohexane conversion and adipic acid selectivity are 25% and 26%, respectively. Although these methods may avoid the serious acid corrosion, the cyclohexane conversion rate and adipic acid selectivity are low, and there are still difficulty of separating the product from the solvent and the like.

In over ten years, studies of producing adipic acid by using molecular oxygen as the oxidant were focused on solvent free conditions. Raja, Sankar and Thomas (J. am. Chem Soc., 1999) reported the study on the catalytic oxidation of cyclohexane in solvent free system using aluminophosphate molecular sieve supported transition metals Fe, Mn, Co, etc. as catalyst, wherein the catalytic effect of FeAlPO-5 was best, cyclohexane conversion rate was 19.8%, adipic acid selectivity was 32.3%, and other products primarily comprise cyclohexanol, cyclohexanone and some esters. Yuan et al (Organic Process Research & Development, 2004) reported the study on the synthesis of adipic acid by catalytic oxidation of cyclohexane using metal porphyrins as catalysts under solvent-free conditions, wherein adipic acid yield was 21.4%. Recently, Lü, Ren and Liu (Applied Catalysis A, 2012) proposed catalytic oxidation of cyclohexane to adipic acid in a solvent-free conditions using Anderson-type catalyst $[(C_{18}H_{37})_2N(CH_3)_2]_6Mo_7O_{24}$, wherein hexane conversion and adipic acid selectivity were up to 10.2% and 87.1%, respectively.

For synthesis of nitrocyclohexane, the traditional mixed acid nitration process is mainly used at present, i.e., using nitric-sulfuric acid or nitric acid-acetic anhydride and the like as the nitrating agent to conduct nitration reaction of cyclohexane. The method has poor selectivity and has many side effects such as oxidation, hydrolysis, hydroxylation and so on, and therefore post-treatment process is complicated, a lot of waste acid and waste water containing organic compounds are produced, and the high environmental treatment cost and serious environmental pollution problem are involved, which deviates far from the requirements of clean production.

Since the nitration reaction of alkanes is more complex, and the current market demand for nitrocyclohexane is far less than adipic acid, there is few reports on the research and development of cyclohexane nitration process. The method of directly nitrating alkanes in gas phase using NOx (nitrogen oxides) wherein x>1 as a nitration agent can avoid the use of large amounts of sulfuric acid, the reaction product contains less free acid and is readily separated, and waste emission is less, but it is sometimes accompanied by side reactions of oxidation, and the main byproducts comprise corresponding alcohols, aldehydes, ketones, acids or nitrate esters and nitrite esters, as well as the deep oxidation byproducts $CO_2$ and $H_2O$ and the like. Canadian Patent No. CA710356 (A) and U.S. Patent No. US 3255262 (A) reported a method of synthesizing nitrocyclohexane by cyclohexane gas phase nitration, mainly for improving the yield of nitrocyclohexane, wherein the reaction temperature is 220-375°C, and the residence time is 20-150 seconds. The main problem of these methods is likely to generate a lot of deep oxidation byproducts $CO_2$ and $H_2O$, and the yield is lower based on cyclohexane. Recently, CN101781217A (2010) proposed a method of coproducing nitrocyclohexane and adipic acid by gas phase non-catalytic or gas-solid catalytic nitration-oxidation of $NO_2$ and cyclohexane, for maximizing the yield of two products nitrocyclohexane and adipic acid, but its main product is nitrocyclohexane, adipic acid is less (usually less than 22% selectivity), and further more deep oxidation byproducts $CO_2$ and CO can not be avoided. Hoot and Kobe (Ind. Eng. Chem., 1955) have reported that cyclohexane and $NO_2$ are sealed in a glass tube at a lower temperature (50-90°C) and reacted for some time, and its main product is adipic acid, followed by nitrocyclohexane and other dicarboxylic acids. However, the conversion rate of cyclohexane is lower (about 2.5%), the reaction time is longer, the overall selectivity of the main product (adipic acid and nitrocyclohexane) is lower, and adipic acid selectivity decreases sharply with the increase of temperature. For example, when the reaction temperature is 50°C, the reaction time is over 40 hours, adipic acid selectivity is about 76%, nitrocyclohexane selectivity is about 3%, and the total selectivity of both is about 79%; when the reaction temperature is 80°C, the reaction time is shortened to 3 hours, adipic acid selectivity decrease to about 40%, nitrocyclohexane selectivity rises to about 7%, and the total selectivity of both is about 47%; when the reaction temperature is 90°C, the reaction time is shortened to about 50 minutes, but adipic acid selectivity is almost zero, and nitrocyclohexane selectivity is about 22%.

**Summary of the Invention**

It is an object of the present invention to provide a method of coproducing adipic acid and nitrocyclohexane with high selectivity in one step from cyclohexane by using NOx (1 <x <3, the same below) as nitrification oxidant. Compared to the current adipic acid and nitrocyclohexane industrial production technology using cyclohexane as raw material, said method not only can greatly simplify the production process and equipment, more importantly, it is able to significantly improve resource utilization, reduce environmental pollution, decrease energy consumption and production costs, and is a new way to achieve green synthesis of adipic acid and nitrocyclohexane. The method of the present invention is more suitable for continuous production.

The basic principle and method of the present invention used to achieve the above-said object is described as below.

For general organic compounds, NOx is a kind of nitrating agent, and also a strong oxidant, so they can conduct nitration and oxidation reaction with NOx under certain conditions. For example, cyclohexane can be nitrated by NOx to produce nitrocyclohexane, while cyclohexane may be oxidized to adipic acid. The reaction equations are as follows:

$$C_6H_{12} + \frac{3}{2(x-1)}NO_x \rightarrow C_6H_{11}NO_2 + \frac{1}{2}H_2O + \frac{5-2x}{2(x-1)}NO$$

$$C_6H_{12} + \frac{5}{x-1}NO_x \rightarrow (CH_2)_4(COOH)_2 + H_2O + \frac{5}{x-1}NO$$

Since these two reactions generate water, there are reactions of producing nitrate or nitrite and the like, such as:

$$3NO_2 + H_2O \rightarrow 2HNO_3 + NO$$

$$N_2O_5 + H_2O \rightarrow 2HNO_3$$

$$3N_2O3 + H_2O \rightarrow 2HNO_3 + 4NO$$

In addition to the above reactions, some other side reactions may also occur, which produce oxidation by-products such as alcohols, ketones, acids, esters and the like, and even deep oxidation products CO and $CO_2$. If the reaction is not controlled appropriately, it could seriously affect the yield of the main products. However, NO produced in the reaction may be recovered, and recycled after mixing with a certain amount of $O_2$:

$$NO + \frac{x-1}{2}O_2 \longrightarrow NO_x$$

Since the reaction of NO and $O_2$ is a spontaneous reaction, from the above point of view of preparing adipic acid, it is a green chemical process of introducing molecular oxygen as an oxidant by using NO as the carrier; likewise, preparation of nitrocyclohexane as described above is also a green chemical process.

Thus, the method of the present invention is to coproduce adipic acid and nitrocyclohexane in one step from cyclohexane by using nitrogen oxides NOx as the oxidant and nitrating agent, with high selectivity and adjustable ratio of adipic acid and nitrocyclohexane, by means of reaction conditions such as reaction temperature, reaction pressure, ratio of reactants, catalyst or inducing agent as control means. NO generated by the reaction is recovered, and used for producing NOx after mixing with a certain amount of $O_2$ and then recycled. The main role of the catalyst or inducing agent is to improve the conversion rate of cyclohexane and NOx utilization, and adjust the proportion of adipic acid and nitrocyclohexane auxiliarily.

According to a first embodiment of the present invention, provided is a method for coproducing adipic acid and nitrocyclohexane from cyclohexane with high selectivity in one step, wherein: cyclohexane is oxidized and nitrated simultaneously using NOx (1<x <3, the same below) as the oxidant and nitrating agent, to coproduce adipic acid and nitrocyclohexane with high selectivity, and by adjusting the cyclohexane conversion conditions, the proportion of adipic acid and nitrocyclohexane may be regulated, adipic acid selectivity can be adjusted in the range of 5% -95%.

In general, the method is carried out in a production apparatus, wherein the apparatus includes a cyclohexane conversion system, a NO oxidation system, a phase separation system, a light phase material separation system and a heavy phase material separation system, the oxidation and nitration reactions are carried out in the cyclohexane conversion system, oxidation of NO produced in above-said reactions is carried out in the NO oxidation system, and the resulting reaction mixture obtained after oxidation and nitration is separated by the phase separation system to obtain light phase material and heavy phase material.

In the present invention, oxidation and nitration of cyclohexane by NOx are carried out in the liquid phase or gas-liquid phase in the conversion system such as a closed reactor (non-tubular reactor such as autoclave or tubular reactor).

Generally, the conversion reaction temperature of the cyclohexane conversion system is 0-150°C, preferably 5-100°C; the reaction pressure of the conversion system is atmospheric pressure -10 MPa, preferably atmospheric pressure -3 MPa; the molar ratio of the reactant cyclohexane and NOx is 0.1-20, preferably 0.2-10; and NOx is nitrogen oxides of $1 < x < 3$, such as $NO_2$, $N_2O_4$, $N_2O_3$, $N_2O_5$, etc., or mixtures thereof, preferably $1.5 < x < 3$.

In the method described above, the reaction product discharged from the cyclohexane conversion system can be separated into light and heavy phase materials by the phase separation system; wherein the light phase material mainly comprises unconverted cyclohexane and nitrocyclohexane dissolved in said cyclohexane, and after separating the unconverted cyclohexane therefrom by the light phase material separation system, nitrocylcohexane crude product may be obtained, and the separated cyclohexane can be recyled; and the heavy phase material mainly comprises adipic acid crystals produced by the reaction, the solid catalyst, and a small amount of acidic aqueous phase, which may obtain adipic acid crude product by the heavy phase material separation system.

Preferably, the gaseous product NO from the cyclohexane conversion system is converted to NOx ($1 < x < 3$) by the NO oxidation system and then recycled; in order to prevent accumulation of trace amounts of non-condensable gases in the system, small amount of gaseous product is treated as off-gas.

Preferably, a catalyst or inducing agent may be added to the cyclohexane conversion system, for improving the conversion rate of cyclohexane and the total selectivity, and auxiliarily adjusting the proportion of the products adipic acid and nitrocyclohexane; when using a solid catalyst, the mass ratio of it and cyclohexane is 0-0.2; when using a liquid catalyst or inducing agent, the mass ratio of it with cyclohexane is 0-0.1.

Typically, the catalyst or inducing agent is vanadium phosphorous oxide complexes, imide-type compounds, molecular sieves, solid acids, metal oxides, organic or inorganic acid salts, Salen transition metal-type catalysts, heteropolyacids, peroxides and alcohols, ketones, aldehydes, esters and the like; light phase material from the phase separation system can also be used as the inducing agent.

Preferably, the solid catalyst is separated from the heavy phase material separation system and directly or indirectly recycled to the cyclohexane conversion system.

Additionally, the method of the present invention is to coproduce adipic acid and nitrocyclohexane in one step from cyclohexane by liquid phase or gas-liquid phase reaction using nitrogen oxides NOx as the oxidant and nitrating agent, with high selectivity and adjustable ratio of adipic acid and nitrocyclohexane, by means of reaction conditions such as reaction temperature, reaction pressure, ratio of reactants, catalyst or inducing agent as control means. NO generated by the reaction is recovered, and used for producing NOx after mixing with a certain amount of $O_2$ and then recycled. The main role of the catalyst or inducing agent is to improve the conversion rate of cyclohexane and utilization of NOx, and adjust the proportion of adipic acid and nitrocyclohexane auxiliarily.

According to a second embodiment of the present invention, provided is a method of coproducing adipic acid and nitrocyclohexane from cyclohexane, the method comprising the steps of:

(a) Reaction: cyclohexane is oxidized and nitrated by NOx in a reactor in liquid phase or gas-liquid phase, in the presence or absence of a catalyst or inducing agent, to obtain a reaction mixture containing nitrocyclohexane and adipic acid, wherein NOx is any one or more of nitrogen oxides satisfying $1 < x < 3$.

Preferably, the above-said method further comprises:

(b) Phase separation: the reaction mixture obtained in step (a) is separated into two phases i.e. light and heavy phases by phase separation (e.g. by standing, settling, or centrifugation), wherein the light phase comprises nitrocyclohexane, unreacted cyclohexane and optional inducing agent, and the heavy phase comprises acidic water phase produced by the reaction, adipic acid crystals and optional catalyst. The upper layer light phase is also known as the oil phase, the lower layer heavy phase is also known as aqueous phase.

More preferably, the above-said method further comprises:

(c) Secondary separation: (c1) the unreacted cyclohexane and optional inducing agent are separated from the light phase obtained in step (b), to obtain crude nitrocyclohexane containing a small amount of by-product, and/or (c2) the crude adipic acid crystals and optional catalyst are separated from the heavy phase obtained in step (b), and the remaining acidic water phase is post-treated in order to recover nitric acid and a small amount of acidic byproducts.

Preferably, the above-said method further comprises:

(d) Recycling: the gas phase obtained in the reaction of (a) (e.g., during the reaction or after completion of the reaction) is supplied to the NO oxidation reactor to react with $O_2$, such that NO is converted to NOx (1 <x <3) for recycling (e.g., recycled to the reactor or stored in a vessel for using in the next batch reaction). In addition, the light phase from (b) contains the inducing agent and small amounts of by-products having inducing action such as alcohols, ketones, esters and the like, and a small amount of light phase is recycled directly as the inducing agent. Further, the catalyst separated in (c) is directly or indirectly (i.e., used after purification or regeneration) recycled.

In the present application, "Optional" or "optionally" means that the subsequently described event or circumstance can or cannot occur.

The reactor used in step (a) of the present application is generally a closed reactor, preferably non-tubular reactor, more preferably an autoclave reactor or high pressure reaction kettle. Preferably, the above described reactor is a closed reactor (non-tubular reactor) such as an autoclave reactor or autoclave, or two or three or four or five or six or seven or eight or more closed reactors such as autoclaves or high pressure reaction kettles in series. Alternatively, the reactor used in step (a) is a closed reactor, for example a tubular reactor. The above reactor is preferably manufactured from stainless steel. The reactor described herein is preferably a tubular reactor or column reactor, and can also be a single tank reactor or tanks-in-series reactor and so on.

Preferably, a mixing device (e.g., paddle or stir bar) is used for mixing in the above-said reactor, for example, for non-tubular reactor, a dynamic mixing device such as a stirring blade or a stirring rod is used, or for the tubular reactor, a static mixing device or a static mixer is used.

When the reactor used in step (a) is one reactor, the method of the present invention can be carried out in a batch mode. When the reactor used in step (a) is two or three or four or five or six or seven or eight or more reactors (such as autoclaves or high pressure reaction kettles) in series, or a tubular reactor, the method of the present invention can be conducted in semi-continuous or continuous manner.

In the method described above, the reaction temperature of the step (a) is 0-150°C, preferably 5-140°C, preferably 10-130°C, preferably 20-120°C, preferably 30-110°C, preferably 40-100 °C, preferably 45-90 °C or 40-95 °C, preferably 50-80 °C or 60-85 °C, more preferably 55-75°C or 65-82°C, still more preferably 58-70°C or 70-80°C.

In the method described above, the molar ratio of cyclohexane and NOx in said step (a) is 0.1-20: 1, preferably 0.15-15: 1, preferably 0.2-10: 1, preferably 0.25-8: 1, preferably 0.3-6: 1, preferably 0.35-4: 1, preferably 0.37-2: 1, preferably 0.40-1: 1, preferably 0.42-0.95: 1, more preferably 0.45-0.75: 1, more preferably 0.50 - 0.70 : 1, or preferably 0.15-0.30: 1, e.g. 0.20: 1. In said step (a), NOx is nitrogen oxides of 1.5 <x <3, preferably $NO_2$, $N_2O_4$, $N_2O_3$, $N_2O_5$, or the mixture of any two, three or four thereof.

In the above described method, the mass ratio of the catalyst and cyclohexane is 0-0.30: 1, preferably 0.005-0.27: 1, preferably 0.01-0.25: 1, preferably 0.05-0.23: 1, preferably 0.08-0.22: 1, preferably 0.10-0.20: 1, and/or the mass ratio of the inducing agent and cyclohexane is 0-0.25: 1, preferably 0.003-0.23: 1, preferably 0.005-0.20: 1, preferably 0.008-0.15: 1, preferably 0.01-0.12: 1, preferably 0.02-0.11: 1, preferably 0.03-0.10: 1.

In the above described method, the reaction of said step (a) is carried out under 1 atmospheric pressure (i.e. 0.10133MPa) -10 MPa, preferably 1.12 atm -5 MPa, preferably 1.15 atm-4MPa, more preferably 1.2 atm -3MPa, further preferably 1.25 atm-2.5MPa, more preferably 0.5-1.5MPa, 0.8-1.3MPa, 0.9-1.25MPa, 1.09-1.20MPa, e.g., under a pressure of 1.18 or 1.13MPa.

Preferably, when the reaction of step (a) is carried out at 40-95 °C, the pressure is generally between 1 atm to 1.3MPa; when the reaction of step (a) is carried out at 50-85°C, the pressure is generally between 1.5 atmospheric pressure and 1.25MPa; when the reaction of step (a) is carried out at 60-80°C, the pressure is generally between 0.6 - 1.18MPa (such as 0.7 or 0.8 or 0.9 or 1.13MPa); or when the reaction of step (a) is carried out at 60-75°C, the pressure is generally between 0.6 - 1.1MPa (such as 0.7 or 0.8MPa); or when the reaction step (a) is carried out at 70-80 °C, the pressure is generally between 1.08 - 1.14MPa (for example 1.13MPa).

In the above described method, the reaction time (or residence time) of said step (a) is usually 0.5-30 hours, preferably 1-28 hours (e.g., 15, 20, 24, 26h), preferably 1.5-25 hours, preferably 2.0-20 hours, preferably 2.5-18 hours, e.g. 3, 4, 5, 6, 7, 8, 9, 10, 11, 12, 13, 14, 15, 16 hours.

When the method of the present invention is conducted in a semi-continuous or continuous manner, the reaction time of step (a) is the total residence time in two or three or four or five or six or seven or eight or more reactors (such as autoclaves or high pressure reaction kettles) in series or in the tubular reactor.

Preferably, in the method of the present invention, "cyclohexane is oxidized and nitrated by NOx in a reactor in liquid phase or gas-liquid phase" refers to, mixing liquid NOx with cyclohexane (for example, dissolving liquid NOx in cyclohexane) and conducting oxidation and nitration reaction at elevated temperature and under stirring or well mixing. More preferably, in the method of the present invention, "cyclohexane is oxidized and nitrated by NOx in a reactor in liquid phase or gas-liquid phase" refers to, mixing liquid NOx with cyclohexane (for example, dissolving liquid NOx in cyclohexane) and conducting oxidation and nitration reaction at elevated temperature and under stirring by a stirring device or well mixing by a static mixing device.

Said "gas-liquid phase" described in the present invention means that, the liquid reaction mixture is present in the reactor, while a gas phase is present in the headspace of the reaction mixture or above liquid level (containing or mainly containing NO gas generated by the reaction).

Preferably, in the above step (a), the liquid NOx is dissolved in cyclohexane at temperature of less than 20°C, preferably less than 15°C (e.g., less than 5°C) to form a reaction mixture. Preferably, the resulting reaction mixture is reacted in a closed reactor at elevated reaction temperatures (e.g., above-said reaction temperature). The reaction of step (a) is more preferably carried out at the condition of sufficient mixing or stirring.

Preferably, the reaction of the above-described step (a) is performed as follows: in the presence or absence of a catalyst or an inducing agent, NOx is dissolved in cyclohexane at a temperature less than 20°C, preferably lower than 15°C and then oxidation and nitration reaction is conducted at an elevated temperature (such as above-described reaction temperature) (preferably, at elevated pressure, and/or with mixing or stirring) in liquid phase or gas-liquid phase in a reactor, to obtain a reaction mixture containing adipic acid and nitrocyclohexane, wherein NOx is any one or more of nitrogen oxides satisfying $1 < x < 3$.

The present inventors have found that a lot of substances can play a catalytic or induced effect, and different catalysts or inducing agents can adjust the ratio of adipic acid and nitrocyclohexane of the reaction product.

In the method described above, the catalyst is one or more selected from the following group of substances: vanadium phosphorous oxide complexes such as M-VPO or M-AIVPO (where M is a transition metal, such as Mn, Cu, Co, Ni, or Cr); imide compounds such as N-hydroxy phthalimide, N,N'-dihydroxy pyromellitic diimide or N-hydroxy-1,8-naphthalimide (which preferably are combined with 9,10-anthraquinone, 1-aminoanthraquinone or 2-aminoanthraquinone as the imide compounds adjuvants); transition metal (e.g. Mn, Cu, Co, Ni, or Cr) acetylacetonate complex catalysts; zeolites or molecular sieves, such as HZSM-5 molecular sieve, H-Y molecular sieve, $\beta$- zeolite, titanium silicalite-1(TS-1) or MAIPO-5 molecular sieve (where M is Fe, Mn, Mg, Co or Cu); solid acid such as sulfonic acid resin, sulfuric acid/silica gel, phosphoric acid/silica gel and $SO_4^{2-}/[TiO_2(4)-MoO_3(1)]$ or $SO_4^{2-}/ZrO_2-Ce_2O_3$; metal oxides such as $TiO_2$, $V_2O_5$, $\gamma-Al_2O_3$, $ZrO_2$, NiO, CrO, $MnO_2$, CuO, $Ce_2O_3$, $WO_3$ or $Co_3O_4/SiO_2-Al_2O_3$; organic or inorganic acid salts, such as acetates of transition metals (e.g., manganese acetate, cobalt acetate, copper acetate), transition metal naphthenates (e.g. cobalt naphthenate), transition metal sulfates (e.g., manganese sulfate, cobalt sulfate, nickel sulfate, copper sulfate or $Fe_2(SO_4)_3$), hydrochloride salt of transition metal (such as cobalt chloride, cupric chloride or zinc chloride); Salen transition metal-based catalysts such as bis(salicylidene)ethylene diamine cobalt (Cosalen), Cosalen/NaY, Cosalen/AlPO-5 or Cosalen/MCM-41; heteropolyacids such as HPAs or phosphotungstic acid; or the inducing agent is one or more selected from the group of substances: peroxides such as benzoyl peroxide, hydrogen peroxide or tert-butyl hydroperoxide, and alcohols, ketones, aldehydes or esters, such as: cyclohexanone, methyl ethyl ketone, acetone; cyclohexanol, pentanol, butanol, propanol, ethanol, methanol; butyraldehyde, propionaldehyde, acetaldehyde; ethyl acetate, n-butyl acetate, ethyl nitrate.

Preferably, the catalyst is vanadium-phosphorus oxide complexes such as M-VPO or M-AIVPO, wherein M is a transition metal, such as Mn, Cu, Co, Ni, or Cr.

In the present invention, NOx is dissolved in cyclohexane (e.g., at a lower temperature such as below 20°C, preferably below 15°C or below 5°C) to form a liquid reaction mixture before the reaction or at the start of the reaction, and then reacted in a closed reactor at an elevated temperature in the form of the liquid mixture, particularly mixed or stirred sufficiently during the reaction or at the same time of the reaction. The present inventors have unexpectedly found that adipic acid is obtained by the method of the present invention in very high conversion rate and selectivity.

By the method described in the present invention, single-pass conversion rate of cyclohexane may be up to 20-60%, preferably 30-50%, the total selectivity of main products adipic acid and nitrocyclohexane may be up to 98% (or 99%) - 99.9% or even 100%. Ratio of the two products is adjustable, and adipic acid selectivity is generally in the range of 60-90%, or 63-85%, e.g., up to 70%, 80% or 85% or 90% (selectivity is based on cyclohexane). Adipic acid purity may be above 98wt%.

In the present application, the pressure refers to the absolute pressure (unit: atm or MPa).

The method of the present invention can be carried out in a batch, semi-continuous or continuous manner.

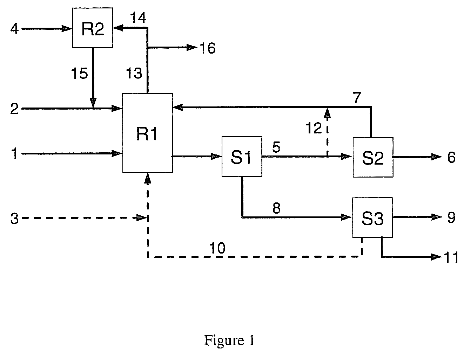

The method of the present invention will be described below with reference to Figure 1. Showed is a batch process in Figure 1, but it can also be a continuous process, and is more suitable for continuous production.

(I) The apparatus of method the present invention primarily consists of a cyclohexane conversion system (R1), a NO oxidation system (R2), a phase separation system (S1), a light phase material separation system (S2) and a heavy phase material separation system (S3).

(II) Oxidation and nitration reaction of cyclohexane (1) and NOx (2) is carried out in the cyclohexane conversion system (R1), to produce adipic acid and nitrocyclohexane primarily. The reaction temperature of the conversion system is at 0-150°C, preferably at a temperature of 5-100 °C; the reaction pressure of the conversion system is atmospheric pressure~10 MPa, preferably atmospheric pressure~3 MPa; the molar ratio of cyclohexane (1) and NOx (2) is 0.1-20, preferably 0.2-10; when using a solid catalyst, the reaction system is the liquid-solid two-phase or gas-liquid-solid three-phase, and the mass ratio of the solid catalyst and cyclohexane is 0-0.2; when using a liquid catalyst or inducing agent, the reaction system is a liquid phase or gas-liquid two-phase, and the mass ratio of the liquid catalyst or inducing agent and cyclohexane is 0-0.1; and the stream (3) is the newly added catalyst or inducing agent.

(III) The reaction product discharged from the cyclohexane conversion system (R1) can be separated into a light phase material (5) and a heavy phase material (8) by the phase separation system (S1). The light phase material (5) mainly is unconverted cyclohexane and the reaction product nitrocyclohexane dissolved therein; and the heavy phase material (8) is mainly the reaction product adipic acid crystals, the solid catalyst, and acidic aqueous phase produced by the reaction.

(IV) By the light phase material separation system (S2), unconverted cyclohexane (7) is separated from the light phase material (5) separating and then returned to the cyclohexane conversion system (R1), and the remaining part is crude nitrocyclohexane (6). Since the light phase material (5) contains inducing agent dissolved therein and a small amount of byproducts having inducing effect such as alcohols, ketones, esters and the like, and thus a small amount of light phase material (12) can be separated and directly supplied to the cyclohexane conversion system for inducing the conversion reaction of cyclohexane.

(V) crude adipic acid crystals (9) and solid catalyst (10) are separated from heavy phase (8) by means of the heavy phase material separation system (S3), and the remaining acidic aqueous phase (11) is post-treated to recover nitric acid and other small amounts of by-products. The isolated solid catalyst (10) may be directly or indirectly recycled.

(VI) Gas (13) produced in the cyclohexane conversion system (R1) will be recycled, wherein most of the gas (14) is conveyed to the NO oxidation reaction system (R2), and reacted with $O_2$ (4), so that NO is converted into NOx (x> 1), which is recycled to the cyclohexane conversion system (15). For a continuous process, in order to prevent trace non-condensable gas from accumulating in the system, it is required to divide a small portion of gas from gas (13) and supplied for exhaust gas treatment (16).

(VII) The main role of the catalyst or inducing agent is to increase cyclohexane conversion rate and total selectivity, and auxiliarily adjust the proportion of the products adipic acid and nitrocyclohexane, and therefore it is determined to add or not add a catalyst or inducing agent according to the actual needs. The present inventors have found that a lot of substances can play the role of the catalysis or induction, which are mainly the following substances or mixtures thereof:

vanadium phosphorous oxide complexes (VPO or AlVPO) or modified catalysts, where M is an oxide of a transition metal such as Mn, Cu, Co, Ni, Cr, etc.; imide compounds such as N-hydroxy phthalimide, N,N'-dihydroxy pyromellitic diimide or N-hydroxy-1,8- naphthalimide, which are combined with anthraquinones such as 9,10-anthraquinone, 1-aminoanthraquinone or 2-aminoanthraquinone as the imide compounds adjuvants; transition metals (e.g. Mn, Cu, Co, Ni, Cr, etc.) acetylacetonate complex catalyst; molecular sieves, such as HZSM-5 molecular sieve, H-Y molecular sieve, β- zeolite, titanium silicalite-1(TS-1) or MAlPO-5 molecular sieve supported metal, where M is an oxide of Fe, Mn, Mg, Co, Cu, etc.; solid acids catalyst such as sulfonic acid resin, sulfuric acid/silica gel, phosphoric acid/silica gel and $SO_4^{2-}/[TiO_2(4)-MoO_3(1)]$, $SO_4^{2-}/ZrO_2-Ce_2O_3$, etc.; metal oxides catalyst such as $TiO_2$, $V_2O_5$, $\gamma$-$Al_2O_3$, $ZrO_2$, NiO, CrO, $MnO_2$, CuO, $Ce_2O_3$, $WO_3$, $Co_3O_4/SiO_2$-$Al_2O_3$, etc.; organic or inorganic acid salt catalysts, such as acetates of transition metals, e.g., manganese acetate, cobalt acetate, copper acetate, etc., cobalt naphthenate, transition metal sulfates e.g., manganese sulfate, cobalt sulfate, nickel sulfate, copper sulfate, $Fe_2(SO_4)_3$), etc., and hydrochloride salt of transition metal such as cobalt chloride, cupric chloride, zinc chloride, etc.; Salen transition metal-based catalysts such as bis(salicylidene)ethylene diamine cobalt (Cosalen) and composite catalysts thereof, such as Cosalen/NaY, Cosalen/AlPO-5, Cosalen/MCM-41, etc.; heteropolyacids catalyst such as HPAs, phosphotungstic acid, etc.; peroxides such as benzoyl peroxide, hydrogen peroxide or tert-butyl hydroperoxide, and alcohols, ketones such as cyclohexanone, methyl ethyl ketone, acetone, and the like; alcohols such as cyclohexanol, amyl alcohol, butanol, propanol, ethanol, methanol and the like; aldehydes such as butyraldehyde, propionaldehyde, acetaldehyde and the like; esters, such as ethyl acetate, n-butyl acetate, ethyl nitrate and the like.

The conversion system according to the above-described process may be a tubular reactor, a tower reactor, etc., can also be a single tank reactor or tanks-in-series reactor, and so on.

By the method described in the present invention, single-pass conversion rate of cyclohexane may be up to 20-70%, the total selectivity of main products adipic acid and nitrocyclohexane may be more than 95%. Ratio of the two products is adjustable, and adipic acid selectivity can be over 85%.

Compared to the exsiting adipic acid and nitrocyclohexane industrial production technology, the one-step method of coproducing adipic acid and nitrocyclohexane from cyclohexane of the present invention not only can greatly simplify the production process and equipment, more importantly, it can greatly improve the conversion of cyclohexane and the selectivity of adipic acid and nitrocyclohexane, significantly reduce waste generation and environmental pollution from the source, and significantly save resources, reduce energy consumption and lower production costs. In particular, it can obtain high value of adipic acid unexpectedly in a relatively simple manner with high selectivity.

**Brief Description of the Drawings**

Figure 1 is a flow chart of the method for coproducing adipic acid and nitrocyclohexane by nitration-oxidation of cyclohexane in one step.

**The Mode of carrying out the Invention**

The following examples are intended to illustrate the invention and not to limit the present invention. Described in the Examples is a batch process, but the continuous process is not limited, and the continuous method is more suitable for continuous production.

Example 1:

5.04 g of cyclohexane, 13.80 g of liquid $NO_2$ and 0.54 g of Cr-AlVPO catalyst are added in a 0.2 L autoclave, at the molar ratio of cyclohexane and $NO_2$ of 0.2: 1 and the ratio by mass of cyclohexane and catalyst of 1: 0.1, and reacted at 80°C and 2.0 MPa under stirring for 24 h. The reaction mixture is allowed to stand and cool for layering, and the upper layer is the oil phase, mainly comprising unreacted cyclohexane and the product nitrocyclohexane, and the lower layer is the mixture of mainly adipic acid crystals, the solid catalyst, water produced by the reaction and the like. The whole mixture is filtered and the filter cake is washed respectively with a specified amount of cyclohexane and distilled water. The collected filtrate and washings were separated into an oil phase and a water phase, and the resulting oil phase and aqueous phase are metered separately. The composition of the oil phase is quantitatively determined by gas chromatography internal standard method, and the composition of the aqueous phase is determined quantitatively by liquid chromatography external standard method. The filter cake is dissolved in a specified amount of methanol, the catalyst solid is filtered and metered, and the resulting filtrate is quantified by liquid chromatography external standard method. According to the material balance, the conversion rate of cyclohexane is 60.3%, the selectivity of adipic acid is 84.4%, the selectivity of nitrocyclohexane is 11.3%, and the sum of the two selectivities is 95.7%.

Example 2: Example 1 is repeated, but the reaction temperature is 100°C, and the reaction pressure is 2.9 MPa. As a result, the conversion rate of cyclohexane is 75.4%, the selectivity of adipic acid is 81.7%, the selectivity of nitrocyclohexane is 11.5%, and the sum of the two selectivities is 93.2%.

Example 3: Example 1 is repeated, but 8.40 g of cyclohexane and 4.60 g of liquid $NO_2$ are added at the molar ratio of cyclohexane and $NO_2$ of 1: 1.0, the catalyst is VPO, and the reaction temperature is 60°C and the reaction pressure is 0.6 MPa. As a result, the conversion rate of cyclohexane is 11.1%, the selectivity of adipic acid is 80.6%, the selectivity of nitrocyclohexane is 11.9%, and the sum of the two selectivities is 92.5%.

Example 4: Example 1 is repeated, but no catalyst is added. As a result, the conversion rate of cyclohexane is 18.6%, the selectivity of adipic acid is 63.1%, the selectivity of nitrocyclohexane is 23.2%, and the sum of the two selectivities is 86.3%.

Example 5: Example 1 is repeated, but 19.6 g of cyclohexane and 3.58 g of liquid $NO_2$ are added at the molar ratio of cyclohexane and $NO_2$ of 3: 1, and no catalyst is added. As a result, the conversion rate of cyclohexane is 9.60%, the selectivity of adipic acid is 53.2%, the selectivity of nitrocyclohexane is 32.0%, and the sum of the two selectivities is 85.2%.

Example 6: Example 1 is repeated, but different catalysts are used. The conversion rate of cyclohexane, the selectivity of adipic acid, the selectivity of nitrocyclohexane and the like are shown in the following table.

| No. | catalyst | Cylcohexane conversion/% | selectivity/% | | |
| --- | --- | --- | --- | --- | --- |
| | | | Adipic acid | nitrohexane | total |
| 1 | Ni-AlVPO | 46.0 | 72.7 | 25.3 | 98.0 |
| 2 | $V_2O_5$ | 58.5 | 82.7 | 12.7 | 95.4 |
| 3 | $Fe_2(SO_4)_3$ | 54.3 | 80.5 | 13.0 | 93.5 |
| 4 | NiO | 55.8 | 80.1 | 13.1 | 93.2 |

(continued)

| No. | catalyst | Cylcohexane conversion/% | selectivity/% | | |
|-----|----------|--------------------------|---------------|---|---|
| | | | Adipic acid | nitrohexane | total |
| 5 | 0.5% Aw/Al$_2$O$_3$ | 53.2 | 79.7 | 14.4 | 94.1 |
| 6 | NHPI | 50.7 | 80.5 | 13.7 | 94.2 |
| 7 | Copper acetylacetone | 48.2 | 80.5 | 13.1 | 93.6 |
| 8 | FeAlPO | 52.1 | 81.4 | 13.3 | 94.7 |
| 9 | sulfuric acid/silica gel | 49.5 | 80.4 | 13.3 | 93.7 |
| 10 | titanium silicalite TS-1 | 47.8 | 81.7 | 12.9 | 94.6 |
| 11 | titanium silicalite Cosalen/MCM-41 | 49.1 | 82.1 | 12.2 | 94.3 |
| 12 | SO$_{42}$-/ZrO$_2$-Ce$_2$O$_3$ | 54.1 | 82.4 | 12.6 | 95.0 |
| 13 | phosphotungstic acid | 49.9 | 79.5 | 14.0 | 93.5 |

Example 7: Example 1 is repeated, but 0.5% of the inducing agent cyclohexanone is added based on the mass of cyclohexane, and the reaction temperature is 80 °C and the reaction pressure is 1.9 MPa. As a result, the conversion rate of cyclohexane is 55.3%, the selectivity of adipic acid is 81.7%, the selectivity of nitrocyclohexane is 12.6%, and the sum of the two selectivities is 94.3%.

Example 8: Example 1 is repeated, but 555.3 g of liquid NO$_2$, 202.0 g of cyclohexane, and 4.0 g of Ni-AlVPO catalyst are added in a 5.0 L autoclave. After standing for cooling, the gaseous components is sampled for analysis, and then metered as about 429.3 g through the exhaust gas measurement method, which mainly comprise NO$_2$ (215.3 g), NO (213.7 g) and trace amounts of cyclohexane and CO$_2$. After discharged gaseous components, all liquid and solid materials are metered, and then suction-filtered for solid-liquid separation to obtain 133.1 g filtrate and 193.5 g cake. The filtrate is separated into 98.4g organic phase and 34.7g aqueous phase, wherein the organic phase mainly contains cyclohexane (78.5 wt%), nitrocyclohexane (20.8 wt%), as well as a small amount of cyclohexyl nitrate (0.32 wt%) and other oxidation products (0.38 wt%), and the aqueous phase mainly contains nitric acid (19.7 wt%) and a small amount of C4-6 dicarboxylic acid (2.9 wt%) and C4-5 dicarboxylic acid anhydride (2.1 wt%). The filter cake is firstly dissolved in a specified amount of methanol, and then filtered to separate the solid catalyst. The filtrate is metered and sampled for analysis, which in addition to methanol contains mainly adipic acid (96.3 wt%) and a small amount of succinic acid and glutaric acid (3.5 wt%) as well as succinic anhydride and glutaric anhydride (0.2 wt%). The methanol solution is distilled under reduced pressure, washed with water and dried to give adipic acid product having 99.6% of purity. According to the material balance, the conversion rate of cyclohexane is 61.46%, the selectivity of adipic acid is 84.6%, and the selectivity of nitrocyclohexane is 10.8%.

Example 9: Firstly, liquid NO$_2$ is dissolved in cold (below 15°C) cyclohexane to formulate a homogeneous solution, wherein the molar ratio of cyclohexane and NO$_2$ is 1: 1. The resulting solution is transferred to a stainless steel autoclave, followed by addition of a certain amount of the catalyst Cr-AlVPO, wherein the mass ratio of cyclohexane and the catalyst is 1: 0.1. The autoclave is sealed and heated with stirring to conduct oxidation reaction. The reaction temperature is 60 °C, the reaction pressure is 0.8MPa (gauge pressure), and the reaction time is 24 h. After completion of the reaction, the reaction mixture is allowed to stand for cooling to room temperature, the gas phase containing NO in the headspace of the reactor is directly introduced to an oxidation reactor and oxidized with pure O$_2$, wherein NO is oxidized into NOx (1 <x <3). Alternatively, the gas phase is collected in a reservoir for storing. After several batches of reaction, a sufficient amount of the gas phase is collected and then introduced into the oxidation reactor and oxidized using pure O$_2$, wherein NO is oxidized into NOx (1 <x <3) for recycling. The nitration product nitrocyclohexane is dissolved in the oil phase of the upper layer, the lower layer is mainly adipic acid, a small amount of oxidation products and catalysts, and adipic acid is deposited as crystals. The oil phase is quantitatively analyzed by gas chromatography internal standard method, and the lower layer is dissolved in an organic solvent to separate adipic acid, which is quantified by liquid chromatography external standard method. According to the material balance, the conversion rate of cyclohexane is 11.2%, the selectivity of adipic acid is 80.7%, the selectivity of nitrocyclohexane is 11.9%, and the sum of the two selectivities is 92.6% (all based on cylcohexane).

Example 10: Example 9 is repeated, but the complex oxides catalyst Co-AlVPO is added, the molar ratio of cyclohexane and NO$_2$ is 0.2: 1, the reaction temperature is 100°C, and the reaction pressure is 2 MPa. As a result, the conversion rate of cyclohexane is 85.3%, the selectivity of adipic acid is 80.5%, the selectivity of nitrocyclohexane is 11.4%, and the sum of the two selectivities is 91.9%.

**Claims**

1. A method of coproducing adipic acid and nitrocyclohexane from cyclohexane in high selectivity, the method comprising the steps of:

   (a) Reaction: cyclohexane is oxidized and nitrated by NOx in a reactor in liquid phase or gas-liquid phase, in the presence or absence of a catalyst or inducing agent, to obtain a reaction mixture containing nitrocyclohexane and adipic acid, wherein NOx is any one or more of nitrogen oxides satisfying 1<x <3; and
   (b) Phase separation: the reaction mixture obtained in step (a) is separated into two phases i.e. light and heavy phases by phase separation, wherein the light phase comprises nitrocyclohexane, unreacted cyclohexane and optional inducing agent, and the heavy phase comprises acidic water phase produced by the reaction, adipic acid crystals and optional catalyst.

2. The method according to claim 1, **characterized in that** the said method further comprises:

   (c) Secondary separation: (c1) the unreacted cyclohexane and optional inducing agent are separated from the light phase obtained in step (b), to obtain crude nitrocyclohexane containing a small amount of by-product, and/or (c2) the crude adipic acid crystals and optional catalyst are separated from the heavy phase obtained in step (b), and the remaining acidic water phase is post-treated in order to recover nitric acid and a small amount of acidic byproducts.

3. The method according to claim 1 or 2, **characterized in that** the said method further comprises:

   (d) Recycling: the gas phase obtained in the reaction of (a) is supplied to the NO oxidation reactor to react with $O_2$, such that NO is converted to NOx (1 <x <3) for recycling.

4. The method according to any one of claims 1-3, **characterized in that** the reaction temperature of the step (a) is 0-150°C, preferably 5-140°C, more preferably 40-100°C.

5. The method according to any one of claims 1-4, **characterized in that** the molar ratio of cyclohexane and NOx in said step (a) in 0.1-20: 1, preferably 0.2-10: 1 or preferably 0.15-0.30: 1.

6. The method according to any one of claims 1-5, **characterized in that** the mass ratio of the catalyst and cyclohexane is 0-0.30: 1, preferably 0.005-0.27: 1, and/or the mass ratio of the inducing agent and cyclohexane is 0-0.25: 1, preferably 0.003-0.23: 1.

7. The method according to any one of claims 1-6, **characterized in that** the reaction of said step (a) is carried out under 1 atmospheric pressure -10 MPa, preferably 1.12 atm -5 MPa.

8. The method according to any one of claims 1-7, **characterized in that** the catalyst is one or more selected from the following group of substances: vanadium phosphorous oxide complexes such as M-VPO or M-AlVPO where M is a transition metal; imide compounds; transition metal acetylacetonate complex catalysts; zeolites or molecular sieves; solid acid; metal oxides; organic or inorganic acid salts; Salen transition metal-based catalysts; and heteropolyacids;
   or the inducing agent is one or more selected from the group of substances: peroxides, alcohols, ketones, aldehydes or esters.

9. The method according to any one of claims 1-8, **characterized in that** said method is carried out in a batch, semi-continuous or continuous manner, preferably in continuous manner.

10. The method according to any one of claims 1-9, **characterized in that** the reactor used in step (a) is a closed reactor, and preferably manufactured from stainless steel.

Figure 1

<div align="center">

# INTERNATIONAL SEARCH REPORT

</div>

| International application No. |
|---|
| **PCT/CN2014/081818** |

## A. CLASSIFICATION OF SUBJECT MATTER

C07C 51/275 (2006.01) i; C07C 201/08 (2006.01) i; C07C 55/14 (2006.01) n; C07C 205/05 (2006.01) n

According to International Patent Classification (IPC) or to both national classification and IPC

## B. FIELDS SEARCHED

Minimum documentation searched (classification system followed by classification symbols)

C07C 51/-; C07C 55/-; C07C 201/-; C07C 205/-

Documentation searched other than minimum documentation to the extent that such documents are included in the fields searched

Electronic data base consulted during the international search (name of data base and, where practicable, search terms used)

CNPAT, CNKI, WPI, EPODOC: nitrogen oxide, vaporphase nitration, adipic acid, cyclohexane, nitrate, nitrocyclohexane, nitrogen, oxide, nitrogen dioxide

## C. DOCUMENTS CONSIDERED TO BE RELEVANT

| Category* | Citation of document, with indication, where appropriate, of the relevant passages | Relevant to claim No. |
|---|---|---|
| A | GB 969793 A (MONTEDISON SPA), 16 September 1964 (16.09.1964), the whole document | 1-10 |
| X | CN 101781217 A (XIANGTAN UNIVERSITY), 21 July 2010 (21.07.2010), description, paragraphs 5-6 and 14-15, and embodiments 1-2 | 1-10 |
| PX | CN 103288626 A (XIANGTAN UNIVERSITY), 11 September 2013 (11.09.2013), the whole document | 1-10 |
| A | EP 1099684 A1 (DAICEL CHEM), 16 May 2001 (16.05.2001), the whole document | 1-10 |
| A | CN 1304398 A (DAICEL CHEMICAL INDUSTRIES LTD.), 18 July 2001 (18.07.2001), the whole document | 1-10 |
| A | CN 102172534 A (XIANGTAN UNIVERSITY), 07 September 2011 (07.09.2011), the whole document | 1-10 |
| A | CN 101074198 A (XIANGTAN UNIVERSITY), 21 November 2007 (21.11.2007), the whole document | 1-10 |

☐ Further documents are listed in the continuation of Box C.    ☒ See patent family annex.

| | |
|---|---|
| \*    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 28 September 2014 (28.09.2014) | **10 October 2014 (10.10.2014)** |

| Name and mailing address of the ISA/CN: <br> State Intellectual Property Office of the P. R. China <br> No. 6, Xitucheng Road, Jimenqiao <br> Haidian District, Beijing 100088, China <br> Facsimile No.: (86-10) 62019451 | Authorized officer <br><br> **LI, Yong** <br><br> Telephone No.: (86-10) **62084574** |

Form PCT/ISA/210 (second sheet) (July 2009)

## INTERNATIONAL SEARCH REPORT
### Information on patent family members

| International application No. |
| --- |
| **PCT/CN2014/081818** |

| Patent Documents referred in the Report | Publication Date | Patent Family | Publication Date |
| --- | --- | --- | --- |
| GB 969793 A | 16 September 1964 | ES 272707 A1 | 16 April 1962 |
| | | CH 407082 A | 15 February 1966 |
| | | BE 611138 A1 | 30 March 1962 |
| CN 101781217 A | 21 July 2010 | CN 101781217 B | 29 May 2013 |
| CN 103288626 A | 11 September 2013 | None | |
| EP 1099684 A1 | 16 May 2001 | CN 1304398 A | 18 July 2001 |
| | | KR 20010071919 A | 31 July 2001 |
| | | JP 2000327635 A | 28 November 2000 |
| | | WO 0069803 A1 | 23 November 2000 |
| | | JP 4685996 B2 | 18 May 2011 |
| | | ID 28884 A | 12 July 2001 |
| CN 1304398 A | 18 July 2001 | EP 1099684 A1 | 16 May 2001 |
| | | KR 20010071919 A | 31 July 2001 |
| | | JP 2000327635 A | 28 November 2000 |
| | | WO 0069803 A1 | 23 November 2000 |
| | | JP 4685996 B2 | 18 May 2011 |
| | | ID 28884 A | 12 July 2001 |
| CN 102172534 A | 07 September 2011 | CN 102172534 B | 13 March 2013 |
| CN 101074198 A | 21 November 2007 | None | |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2223493 A
- US 4263453 A
- US 5321157 A
- FR 2722783 A
- FR 2746671 A
- US 0147777 A1
- US 7507856 B2
- US 0095258 A1
- CA 710356 A
- US 3255262 A
- CN 101781217 A

**Non-patent literature cited in the description**

- **RAJA ; SANKAR ; THOMAS.** *J. am. Chem Soc.,* 1999
- **YUAN et al.** *Organic Process Research & Development,* 2004
- **LÜ ; REN ; LIU.** *Applied Catalysis A,* 2012
- **HOOT ; KOBE.** *Ind. Eng. Chem.,* 1955